# EUROPÄISCHE PATENTSCHRIFT

(11) **EP 2 262 438 B1**
(45) Veröffentlichungstag und Bekanntmachung des Hinweises auf die Patenterteilung: **28.08.2013**
(21) Anmeldenummer: 09730359.8
(22) Anmeldetag: 07.04.2009
(51) Int. Cl.: A61B 18/12, A61B 5/04

(54) **CHIRURGISCHES GERÄT MIT NERVTESTEINRICHTUNG**
SURGICAL APPARATUS COMPRISING A NERVE TESTING DEVICE
APPAREIL CHIRURGICAL COMPORTANT UN DISPOSITIF DE TEST DES NERFS

(30) Priorität: 10.04.2008 DE 102008018262
(43) Veröffentlichungstag der Anmeldung: 22.12.2010
(73) Patentinhaber: ERBE Elektromedizin GmbH, 72072 Tübingen (DE)
(72) Erfinder: NOTZ, Jürgen, 72581 Dettingen (DE); BELLER, Jürgen, 72810 Gomaringen (DE)
(74) Vertreter: Bohnenberger, Johannes
(86) Internationale Anmeldenummer: PCT/EP2009/002571
(87) Internationale Veröffentlichungsnummer: WO 2009/124726

(56) Entgegenhaltungen:
- WO-A-00/13599
- WO-A-01/12089
- US-A1- 2002 095 199
- US-A1- 2005 283 148

## Beschreibung

Die Erfindung betrifft ein chirurgisches Gerät zum Testen von Nerven.

In der Hochfrequenzchirurgie (HF-Chirurgie) werden mittels chirurgischer Instrumente, welche an chirurgische Geräte angeschlossen sind, Gewebestrukturen geschnitten bzw. koaguliert. Sind jene Gewebestrukturen von Nerven durchzogen, so kann die Verwendung des chirurgischen Instrumentes zu Schädigungen oder einer Zerstörung der Nerven führen. Werden beispielsweise Nerven im Gesicht verletzt, wird der mimische Ausdruck des Patienten beeinträchtigt, was als unangenehm empfunden wird.

Aus diesem Grund ist es üblich, beim Schneiden bzw. Koagulieren von Gewebe, welches womöglich von Nerven durchzogen ist, an die beabsichtigte Schnittstelle bzw. Koagulationsstelle einen Probestrom zu geben, der eine Stimulation des entsprechenden Muskels bewirkt. Man weiß dann, ob eine bzw. welche Muskelpartie betroffen ist und kann gegebenenfalls den Schnitt anders legen. Für diesen Probestrom muss aber ein Gleichstrom oder ein niederfrequenter Wechselstrom vorhanden sein, welcher in der Regel über separate Nervtesteinrichtungen zur Verfügung gestellt wird.

In diesem Zusammenhang offenbart die US 2006/0184164 A1 ein HF-Chirurgiegerät mit einem chirurgischen Instrument, welches in verschiedenen Modi, nämlich in solchen zum Schneiden, Koagulieren von Gewebe sowie zum Stimulieren betrieben werden kann. Das HF-Chirurgiegerät umfasst dabei eine Gleichstromversorgungseinrichtung, welche einen Gleichstrom zum Stimulieren von Nerven zur Verfügung stellt und einen HF-Generator, welcher hochfrequenten Strom zum Schneiden oder Koagulieren von Gewebe bereitstellt. Als nachteilhaft wird dabei empfunden, dass das HF-Chirurgiegerät aufwändig konstruiert ist und einen großen Platzbedarf aufweist.

Die WO 00/13599 und die WO 00/13600 zeigen ein HF-Chirurgiegerät, an welches ein chirurgisches Instrument zur Behandlung motorischer Nerven mittels hochfrequentem Strom angeschlossen werden kann. Neben der Behandlung der Nerven ist auch deren Stimulation möglich, wobei diese beispielsweise mit demselben chirurgischen Instrument des HF-Chirurgiegeräts, welches auch zur Behandlung der Nerven zum Einsatz kommt, durchgeführt werden kann. Die Energie für die Behandlung des Gewebes wird dabei durch einen HF-Genera-tor bereitgestellt, wohingegen die Energie für die Stimulation des Gewebes durch einen Pulsgenerator zur Verfügung gestellt wird. Auch das HF-Chirurgieinstrument der WO 00/13599 und der WO 00/13600 ist allerdings relativ kompliziert und Platz einnehmend.

Die US 2002/0095199 offenbart ein Chirurgiegerät nach der Präambel von Anspruch 1.

Der Erfindung liegt die Aufgabe zugrunde, ein chirurgisches Gerät aufzuzeigen, welches mit geringerem Aufwand herstellbar und Platz sparend ist. Diese Aufgabe wird durch ein chirurgisches Gerät nach Patentanspruch 1 gelöst.

Insbesondere wird die Aufgabe durch ein chirurgisches Gerät zum Testen von Nerven während einer Operation unter Verwendung eines HF-chirurgischen Generators und eines elektrochirurgischen Instruments gelöst, wobei das chirurgische Gerät eine Wandlereinrichtung zum Wandeln eines hochfrequenten Behandlungsstromes aus dem HF-chirurgischen Generator in einen Nervenstimulationsstrom und einen steuerbaren Umschalter zum wahlweisen Zuführen entweder des Behandlungsstroms oder des Nervenstimulationsstroms zum elektrochirurgischen Instrument umfasst.

Ein wesentlicher Punkt der Erfindung liegt darin, dass die Wandlereinrichtung den in dem chirurgischen Gerät zur Verfügung stehenden hochfrequenten Behandlungsstrom aus dem HF-chirurgischen Generator derartig umwandelt, dass dieser auch zur Stimulation von Nerven ausgenutzt werden kann. Dadurch entfällt die Notwendigkeit eines separaten Gleichstrom- bzw. Niederfrequenzstrom-Generators zur Nervenstimulation, was zur Folge hat, dass das chirurgische Gerät nicht nur Platz sparender als die im Stand der Technik bekannten Geräte ausgelegt ist, sondern auch vereinfacht herstellbar ist. Dadurch können Kosten eingespart werden.

Die Wandlereinrichtung umfasst eine Gleichrichterschaltung zur Erzeugung eines Gleichstroms als Nervenstimulationsstrom. Dadurch kann in besonders einfacher Weise der hochfrequente Behandlungsstrom des HF-chirurgischen Generators in einen zur Stimulation von Nerven geeigneten Strom umgewandelt werden.

In einer bevorzugten Ausführung umfasst die Wandlereinrichtung einen Frequenzteiler zum Erzeugen eines niederfrequenten Stimulationsstroms, was ebenso eine besonders einfache und konstruktiv wenig aufwändige Möglichkeit zur Umwandlung des hochfrequenten Behandlungsstromes des HF-chirurgischen Generators in einen zur Stimulation der Nerven geeigneten Strom darstellt.

Vorzugsweise umfasst die Wandlereinrichtung eine Pulsmodulationseinrichtung zur Erzeugung eines zeitlich definierten Pulses oder zeitlich definierter Pulsfolgen des Nervenstimulationsstroms. Dadurch können geeignete Stimulationsmuster hergestellt werden.

In einer bevorzugten Weiterbildung umfasst die Wandlereinrichtung eine Konstantstromquelle zur Erzeugung einer konstanten Stromstärke des Nervenstimulationsstroms. Diese Weiterbildung ist zur Erzeugung reproduzierbarer Reize geeignet.

Vorzugsweise ist die Konstantstromquelle auf vorbestimmte Stromstärken einstellbar. Dies ermöglicht eine Anpassung der Wandlereinrichtung an die innerhalb des menschlichen Körpers variierenden Gewebe bzw. deren spezifische elektrische Widerstände.

In einer bevorzugten Ausführungsform umfasst die Wandlereinrichtung eine Ansteuereinrichtung, die mit dem HF-chirurgischen Generator diesen derart steuernd verbunden ist, dass Ausgangsstromparameter und Spannungsparameter des HF-chirurgischen Generators zur Erzeugung definierter Nervenstimulationsstrom-Parameter einstellbar sind. Dies ist eine einfache Möglichkeit, definierte Nervenstimulationsstrom-Parameter einzustellen.

Vorzugsweise ist die Wandlereinrichtung in einem gesonderten Gehäuse oder Gehäuseeinschub angeordnet und derart ausgebildet, dass es mit dem chirurgischen Gerät über Steckverbindungen oder dergleichen verbindbar ist. Eine solche Ausbildungsform eignet sich besonders zum Aufrüsten eines bereits vorhandenen chirurgischen Gerätes mit der Wandlereinrichtung.

Nachfolgend wird die Erfindung auch hinsichtlich weiterer Merkmale und Vorteile anhand von Ausführungsbeispielen beschrieben, die anhand der Abbildungen näher erläutert werden. Hierbei zeigen:
- Fig. 1: eine schematische Darstellung eines chirurgischen Gerätes mit angeschlossenem elektrochirurgischen Instrument sowie einem zu behandelnden Gewebeabschnitt,
- Fig. 2: eine alternative, von Fig. 1 abweichende Ausführungsform des chirurgischen Geräts mit dem angeschlossenen elektrochirurgischen Instrument sowie mit dem zu behandelnden Gewebe aus Fig. 1,
- Fig. 3: eine alternative, von Fig. 1 abweichende Ausführungsform der Wandlereinrichtung und
- Fig. 4: eine weitere alternative Ausführungsform der Wandlereinrichtung, zusammen mit dem elektrochirurgischen Instrument und dem Gewebeabschnitt aus den Figuren 1 und 2.

In der nachfolgenden Beschreibung sind für gleiche und gleich wirkende Teile dieselben Bezugsziffern verwendet.

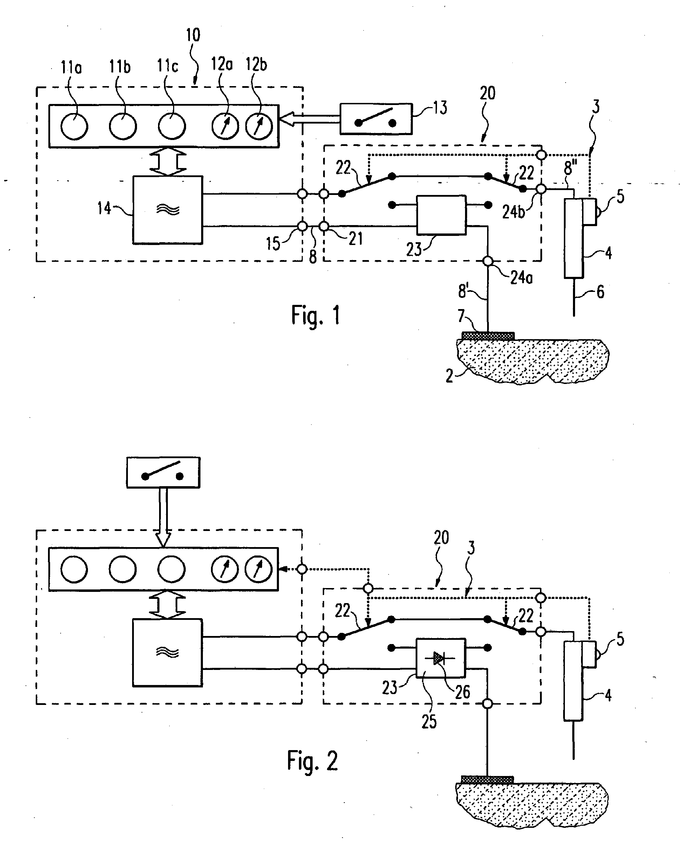

Fig. 1 zeigt ein chirurgisches Gerät mit einer Wandlereinrichtung 20 und einem HF-chirurgischen Generator 10 sowie einem angeschlossenen elektrochirurgischen Instrument 4. Zusätzlich ist ein Gewebeabschnitt 2 gezeigt, welcher mit dem elektrochirurgischen Instrument 4 behandelt bzw. stimuliert werden kann.

Der HF-chirurgische Generator 10 umfasst hier einen HF-Oszillator 14 sowie drei Bedienelemente 11 a bis 11c und zwei Anzeigeelemente 12a, 12b. Über einen Schalter 13 kann der HF-chirurgische Generator 10 gesteuert werden. Der Schalter 13 ist hier außerhalb des HF-chirurgischen Generators 10 angeordnet und mit diesem beispielsweise drahtgebunden oder auch drahtlos verbunden und z. B. als Fußschalter ausgebildet.

Über die Bedienelemente 11 a bis 11c können verschiedene Parameter wie Frequenz, Spannung, Stromstärke, etc., welche den HF-chirurgischen Generator 10 kennzeichnen, eingestellt werden. Die Anzeigeelemente 12a, 12b zeigen diese einstellbaren und weitere Parameter an.

Ein hochfrequenter Behandlungsstrom aus dem HF-chirurgischen Generator wird über Verbindungsleitungen 8 an Wandlereinrichtungseingänge 21 der Wandlereinrichtung 20 geleitet.

Je nach Stellung von steuerbaren Umschaltern 22 passiert der hochfrequente Behandlungsstrom die Wandlereinrichtung 20 entweder im Wesentlichen unverändert (wie bei der Stellung der Umschalter 22 in Fig. 1) oder aufgrund eines Umweges über eine Wandlerschaltung 23 in abgewandelter Form.

Ein Wandlereinrichtungsausgang 24a ist über eine Verbindungsleitung 8' mit einer Neutralelektrode 7, welche mit einem Gewebe 2 verbunden ist, verbunden. Ein Wandlereinrichtungsausgang 24b wiederum ist über eine Verbindungsleitung 8" mit dem elektrochirurgischen Instrument 4 verbunden, welches eine aktive Elektrode 6 umfasst.

Weiterhin weist das elektrochirurgische Instrument 4 ein Bedienelement 5 auf, welches über eine Informationsübertragungsvorrichtung 3 mit den Umschaltern 22, diese steuernd, verbunden ist. Auch diese Verbindung kann drahtgebunden oder auch drahtlos, beispielsweise über eine Infrarot- oder Funkverbindung, konkret ausgeführt sein. Im Falle einer drahtlosen Verbindung weisen dann die Wandlereinrichtung 20 und das elektrochirurgische Instrument 4 entsprechende (nicht in Figuren gezeigt) Empfangs- und Sendeeinrichtungen auf, die die entsprechenden Informationen empfangen bzw. senden.

Über die Informationsübertragungsvorrichtungen 3 können dabei auch weitere Informationen (neben der Ein- bzw. Aus-Information) übertragen werden. So ist es beispielsweise denkbar, dass eine Fehlfunktion der Wandlereinrichtung 20 an ein Anzeigeelement (nicht in Figuren gezeigt) des elektrochirurgischen Instrumentes 4 übertragen wird. Auch kann über das Bedienelement 5 des elektrochirurgischen Instrumentes 4 über verschieb- oder verdrehbare Elemente eine Feineinstellung der Wandlereinrichtung 20, beispielsweise bezüglich verschiedener Parameter wie Frequenz, Spannung, Strom, etc., vorgenommen werden.

Die Wandlerschaltung 23 umfasst eine Gleichrichterschaltung 25 (siehe Fig. 2) und kann auch einen Frequenzteiler (nicht in Figuren gezeigt) umfassen.

In Fig. 2 umfasst die Wandlerschaltung 23 eine Gleichrichterschaltung 25 mit einer Diode 26. Weiterhin ist in Fig. 2 das Bedienelement 5 des elektrochirurgischen Instrumentes 4 über die Informationsübertragungsvorrichtung 3 nicht nur mit den Umschaltern 22 verbunden, sondern auch mit dem HF-chirurgischen Generator 10. Die Ansteuereinrichtung steuert dabei den HF-chirurgischen Generator 10 derart, dass die Ausgangsstromparameter und Spannungsparameter des HF-chirurgischen Generators 10 zur Erzeugung des gewünschten Nervenstimulationsstroms einstellbar sind.

Wie auch bereits aus Fig. 1 zu ersehen, ist auch in Fig. 2 die Gesamtanordnung aus im Wesentlichen zwei Modulen, nämlich dem HF-chirurgischen Generator 10 und der Wandlereinrichtung 20 aufgebaut. Alternativ denkbar ist es natürlich auch, dass die Wandlereinrichtung 20 integraler Bestandteil des HF-chirurgischen Generators 10 ist. Die modulare Ausgestaltung hat allerdings den Vorteil, dass damit ein Nachrüsten handelsüblicher HF-chirurgischer Geräte leichter möglich ist.

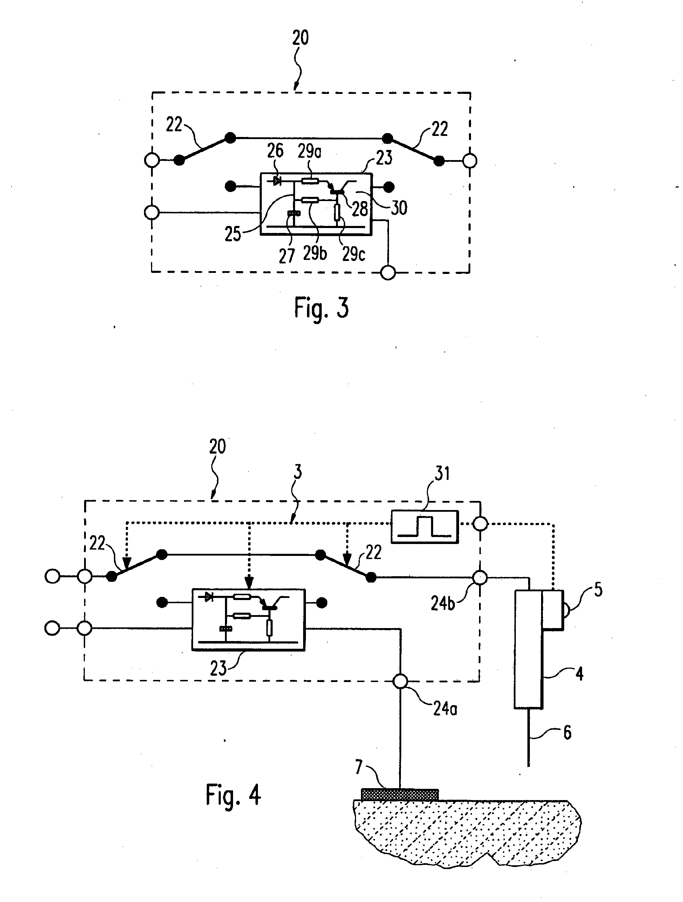

In Fig. 3 ist eine Weiterbildung der Wandlerschaltung 23 aus Fig. 2 zu sehen. Die Gleichrichterschaltung 25 wird hier durch die Diode 26 und eine Kapazität 27 gebildet, während eine Konstantstromquelle 30 in bekannter Art und Weise durch einen Transistor 28 (hier konkret ein pnp-Transistor) und Widerstände 29a bis 29c gebildet wird.

Wie in Fig. 4 gezeigt, kann die Informationsübertragungsvorrichtung 3 auch das Bedienelement 5 des elektrochirurgischen Instrumentes 4 mit der Wandlerschaltung 23 (analog Fig. 3) verbinden, so dass eine Einstellung der Wandlerschaltung 23 über das Bedienelement 5 des elektrochirurgischen Elementes 4 erfolgen kann. Auch können Informationen bzw. Daten der Wandlerschaltung 23 über die Informationsübertragungsvorrichtung 3 an das elektrochirurgische Instrument 4 erfolgen. Insbesondere kann dabei eine Einstellung der Wandlerschaltung 23 bezüglich einer vorbestimmten Stromstärke vorgenommen werden.

In Fig. 4 ist auch zu sehen, dass zwischen dem elektrochirurgischen Instrument 4 und der Wandlerschaltung 23 eine Pulsmodulationseinrichtung 31 angeordnet ist, welche sowohl mit der Wandlerschaltung 23 als auch mit dem elektrochirurgischen Instrument 4 durch die Informationsübertragungsvorrichtung 3 verbunden ist. Die Pulsmodulationseinrichtung 31 dient dabei der Erzeugung eines zeitlich definierten Pulses oder zeitlich definierter Pulsfolgen des Nervenstimulationsstroms. Vorzugsweise führt eine einmalige Betätigung des Bedienelementes 5 zu einem solchen Auslösen des Pulses oder der Pulsfolge, so dass das Gewebe nur über einen bestimmten (kurzen) Zeitraum einem Stimulationsstrom ausgesetzt wird und ein neuerliches Betätigen des Bedienelementes 5 zur Wiedereinstellung eines Schneide- bzw. Koagulationsbetriebes des elektrochirurgischen Instrumentes 4, nicht nötig ist.

An dieser Stelle sei darauf hingewiesen, dass alle oben beschriebenen Teile für sich alleine gesehen und in jeder Kombination, insbesondere die in den Zeichnungen dargestellten Details als erfindungswesentlich beansprucht werden. Abänderungen hiervon sind dem Fachmann geläufig.

### Bezugszeichenliste

- 2: Gewebeabschnitt
- 3: Informationsübertragungsvorrichtung
- 4: elektrochirurgisches Instrument
- 5: Bedienelement
- 6: aktive Elektrode
- 7: Neutralelektrode
- 8: Verbindungsleitung
- 10: HF-chirurgischer Generator
- 11a, 11b, 11c: Bedienelement
- 12a, 12b: Anzeigeelement
- 13: Schalter
- 14: HF-Oszillator
- 20: Wandlereinrichtung
- 21: Wandlereinrichtungseingänge
- 22: Umschalter
- 23: Wandlerschaltung
- 24a, 24b: Wandlereinrichtungsausgänge
- 25: Gleichrichterschaltung
- 26: Diode
- 27: Kapazität
- 28: Transistor
- 29a, 29b, 29c: Widerstand
- 30: Konstantstromquelle
- 31: Pulsmodulationseinrichtung

## Patentansprüche

1. Chirurgisches Gerät zum Testen von Nerven während einer Operation unter Verwendung eines HF-chirurgischen Generators (10) und eines elektrochirurgischen Instruments (4), umfassend
eine Wandlereinrichtung (20) zum Wandeln eines hochfrequenten Behandlungsstromes aus dem HF-chirurgischen Generator (10) in einen Nervenstimulationsstrom;
einen steuerbaren Umschalter (22) zum wahlweisen Zuführen entweder des Behandlungsstroms oder des Nervenstimulationsstroms zum elektrochirurgischen Instrument (4),
**dadurch gekennzeichnet, dass**
die Wandlereinrichtung (20) eine Gleichrichterschaltung (25) zur Erzeugung eines Gleichstroms als Nervenstimulationsstrom umfasst.

2. Chirurgisches Gerät nach einem der vorhergehenden Ansprüche,
**dadurch gekennzeichnet, dass**
die Wandlereinrichtung (20) einen Frequenzteiler zum Erzeugen eines niederfrequenten Stimulationsstroms umfasst.

3. Chirurgisches Gerät nach einem der vorhergehenden Ansprüche,
**dadurch gekennzeichnet, dass**
die Wandlereinrichtung (20) eine Pulsmodulationseinrichtung (31) zur Erzeugung eines zeitlich definierten Pulses oder zeitlich definierter Pulsfolgen des Nervenstimulationsstroms umfasst.

4. Chirurgisches Gerät nach einem der vorhergehenden Ansprüche,
**dadurch gekennzeichnet, dass**
die Wandlereinrichtung (20) eine Konstantstromquelle (30) zur Erzeugung einer konstanten Stromstärke des Nervenstimulationsstroms umfasst.

5. Chirurgisches Gerät nach Anspruch 4,
**dadurch gekennzeichnet, dass**
die Konstantstromquelle (30) auf vorbestimmte Stromstärken einstellbar ist.

6. Chirurgisches Gerät nach einem der vorhergehenden Ansprüche,
**dadurch gekennzeichnet, dass**
die Wandlereinrichtung (20) in einem gesonderten Gehäuse oder Gehäuseeinschub angeordnet und derart ausgebildet ist, dass sie mit dem chirurgischen Gerät über Steckverbindungen oder dergleichen verbindbar ist.

## Claims

1. Surgical apparatus for testing nerves during an operation using an RF-surgical generator (10) and an electrosurgical instrument (4), comprising
a transducer device (20) for converting a radiofrequency treatment current from the RF-surgical generator (10) into a nerve-stimulation current;
a controllable changeover switch (22) for optional return of either the treatment current or the nerve-stimulation current to the electrosurgical instrument (4),
**characterized in that**
the transducer device (20) comprises a rectifier circuit (25) for generating a DC current as nerve-stimulation current.

2. Surgical apparatus according to the preceding claim,
**characterized in that**
the transducer device (20) comprises a frequency splitter for generating a low-frequency stimulation current.

3. Surgical apparatus according to one of the preceding claims,
**characterized in that**
the transducer device (20) comprises a pulse-modulation device (31) for generating a temporally defined pulse or temporally defined pulse sequences of the nerve-stimulation current.

4. Surgical apparatus according to one of the preceding claims,
**characterized in that**
the transducer device (20) comprises a constant-current source (30) for generating a constant current intensity of the nerve-stimulation current.

5. Surgical apparatus according to Claim 4,
**characterized in that**
the constant-current source (30) can be set to predetermined current intensities.

6. Surgical apparatus according to one of the preceding claims,
**characterized in that**
the transducer device (20) is arranged in a separate housing or housing slot and designed in such a way that it can be connected to the surgical apparatus by plug-in connections or the like.

## Revendications

1. Appareil chirurgical pour tester des nerfs pendant une opération en utilisant un générateur HF chirurgical (10) et un instrument électrochirurgical (4), comprenant
un dispositif convertisseur (20) pour convertir un courant de traitement à haute fréquence issu du générateur HF chirurgical (10) en un courant de stimulation des nerfs ;
un inverseur commandable (22) pour acheminer, au choix, soit le courant de traitement, soit le courant de stimulation des nerfs, à l'instrument électrochirurgical (4),
**caractérisé en ce que** le dispositif convertisseur (20) comprend un circuit redresseur (25) pour générer un courant continu en tant que courant de stimulation des nerfs.

2. Appareil chirurgical selon la revendication précédente, **caractérisé en ce que** le dispositif convertisseur (20) comprend un diviseur de fréquence pour générer un courant de stimulation à basse fréquence.

3. Appareil chirurgical selon l'une des revendications précédentes, **caractérisé en ce que** le dispositif convertisseur (20) comprend un dispositif de modulation d'impulsions (31) pour générer une impulsion définie dans le temps ou une série d'impulsions définie dans le temps du courant de stimulation des nerfs.

4. Appareil chirurgical selon l'une des revendications précédentes, **caractérisé en ce que** le dispositif convertisseur (20) comprend une source de courant constant (30) pour générer une intensité de courant constante du courant de stimulation des nerfs.

5. Appareil chirurgical selon la revendication 4, **caractérisé en ce que** la source de courant constant (30) peut être réglée à des intensités de courant prédéfinies.

6. Appareil chirurgical selon l'une des revendications précédentes, **caractérisé en ce que** le dispositif convertisseur (20) est disposé dans un boîtier séparé ou un module enfichable de boîtier et est configuré de telle sorte qu'il peut être relié avec l'appareil chirurgical par des connexions à enfichage ou similaires.
